# EUROPEAN PATENT SPECIFICATION

(11) **EP 3 160 937 B1**
(45) Date of publication and mention of the grant of the patent: **30.12.2020**
(21) Application number: 14896068.5
(22) Date of filing: 03.11.2014
(51) Int. Cl.: C07C 213/00, C07C 215/60, C07C 215/30, C07C 221/00, C07C 225/16

(54) **A NOVEL METHOD FOR SYNTHESIS OF OPTICALLY PURE BETA-AMINO ALCOHOLS**
NEUARTIGES VERFAHREN ZUR SYNTHESE VON OPTISCH REINEN BETA-AMINO-ALKOHOLEN
NOUVEAU PROCÉDÉ DE SYNTHÈSE DE BÊTA-AMINO ALCOOLS OPTIQUEMENT PURS

(30) Priority: 27.06.2014 IN 2090MU2014
(43) Date of publication of application: 03.05.2017
(73) Proprietor: Embio Limited, Mumbai 400076 (IN)
(72) Inventor: JOSHI, Sunil Vaman, Mahad 402309 (IN); BUTALA, Minal Madhukar, Mahad 402301 (IN); GOLLAPUDY, Subrahmanyam, Hyderabad 500033 (IN)
(74) Representative: ABYOO
(86) International application number: PCT/IB2014/065763
(87) International publication number: WO 2015/198108

(56) References cited:
- US-A1- 2005 277 791
- JUN-ICHI IWAO ET AL: "Studies on Alkanolamines. II : Synthesis of N-Methylephedrone and Its Derivatives. Application of the Viogt Reaction", YAKUGAKU ZASSHI = JOURNAL OF THE PHARMACEUTICAL SOCIETY OF JAPAN, vol. 74, no. 5, 1954, pages 551-554, XP055417139, JP ISSN: 0031-6903, DOI: 10.1248/yakushi1947.74.5_551
- MOHANE SELVARAJ COUMAR ET AL: "Identification, SAR Studies, and X-ray Co-crystallographic Analysis of a Novel Furanopyrimidine Aurora Kinase A Inhibitor", CHEMMEDCHEM, vol. 5, no. 2, 2010, pages 255-267, XP055417143, ISSN: 1860-7179, DOI: 10.1002/cmdc.200900339
- 'Thermal rearrangement of ?-hydroxy imines with an ?-allyl or an ?-propargyl substituent' TETRAHEDRON LETTERS vol. 31, 1990, pages 2277 - 2280, XP055246829

## Description

### Technical Field of the Invention

The present invention relates to a novel method for preparation of chiral, non-racemic β-amino alcohols from α-hydroxyketones by first conversion to chiral α-amino ketones by treatment with amines. More particularly, the present invention relates to a method for preparation of norephedrine type compounds from arylacetylcarbinols by reaction with chiral α-(aryl) alkyl amines, followed by reduction to produce the norephedrine type compounds.

### Background of the Invention

Several synthesis method of β-amino alcohol have been reviewed elaborately such as amination of ketones substituted at α-position with a suitable leaving group e.g. halo, tosyloxy etc. followed by reduction of ketone function that give rise to β-Amino alcohols. Some other such methods reported in the state of the art are amination of alcohols substituted at α-position with a suitable leaving group e.g. halo, tosyloxy etc., reduction of α-hydroxy or α-keto oximes, reductive amination of α-hydroxy ketones, reduction of α-azido alcohols or ketones, Reduction of α-amino acids or esters.

β-amino alcohols may exist as pairs of enantiomers and also pairs of diastereomers depending upon the substituents on the carbon atoms bearing the amino and hydroxy groups. The above methods may give rise to enantiomers and diastereomers unless enantioselective synthetic techniques are employed or chiral reduction methods are used to obtain an optically pure compound. There are reports of conversion of α-hydroxy ketones to α-amino ketones by treatment with amines by a reaction called as Voigt reaction, amadori rearrangement etc.

H.Stain (J. Am. Chem. Soc. 51, 269-273, 1929), P.L.Julian et al (J. Am. Chem. Soc.67, 1203-1211, 1945), R.E.Lutz et al (J. Am. Chem. Soc. 70, 2015-2023 (1948) and J. Org. Chem. 21, 49-60 (1956)), R.M.Cowper, T.S.Stevens (J. Chem. Soc. 347-349, 1940) reports conversion of benzoins to α-anilino-deoxy benzoins. Jun-Ichi Iwao et al (J. Pharm Soc. Japan 74, 551-554, 1954) discuss conversion of benzoins and phenylacetylcarbinols to α-anilino- , α-pyrrolidino or α-piperazino deoxybenzoins and propiophenones respectively on treatment with corresponding amines. However, there is no clear understanding of the possible stereo chemical implications of these transformations.

Shun-Ichi Yamada et al (Chem. Communications 1058-1060, 1967) discuss this aspect of the thermal rearrangement. According to them, when the S-3-hydroxy-3-phenylbutan-2-one is treated with benzylamine, it forms imine, which under thermal rearrangement conditions, gave rise to two products, Product A, arising from migration of methyl group and Product B, arising from migration of phenyl group, the latter, apparently in a stereospecific manner. The combined yield of A and B together was only 38%.

Jean-Michele Vatele et al (Tetrahedron Letters 31, 2277-2280, 1990), P.Compain et al (Tetrahedron Letters 36, 4059-4062, 1995; Tetrahedron 52, 6647-6664, 1996) report thermal rearrangements of α-hydroxy imines to give α-amino ketones. In all these cases the hydroxy groups are tertiary and the rearrangement takes place stereo specifically with migration of alkyl groups from the tertiary center with attendant conversion of hydroxy group to keto group. The stereo specificity is controlled by chirality of the tertiary hydroxy center. However, the yields and chiral outcomes are far away from satisfactory.

The prior art EP1512677A1 teaches preparation of α-amino derivatives of aryl alkyl ketones by reaction of α-halo or tosyloxy aryl alkyl ketones with chiral α-(aryl)ethyl amines, whereby the α-(aryl)ethyl amino derivatives of aryl alkyl ketones are prepared as mixtures of diastereomers. The said mixtures of diastereomers are separated by fractional crystallization. There is no evidence of stereoselectivity in this reaction. The said α-amino derivatives of aryl alkyl ketones were further hydrogenated to norephedrine derivatives. The reaction is depicted in scheme 1.

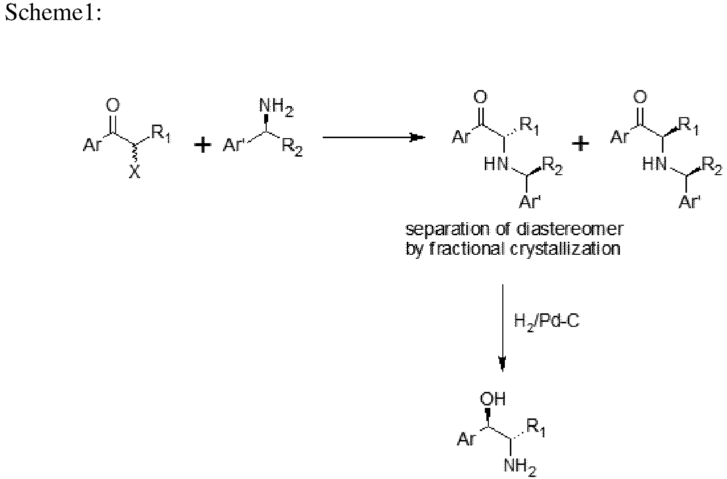

The above mentioned prior arts do not provide a satisfactory method of conversion of α-hydroxy ketone to α-amino ketone in a stereospecific manner. Especially when the α-hydroxy ketone has a secondary hydroxyl group, which may or may not be asymmetric in nature. The present invention seeks to meet this requirement to convert α-hydroxy ketone to α-amino ketone in a stereo selective manner, by reaction with a chiral amine, irrespective of the chirality of hydroxyl center, utilizing the chirality of the amine reactant. This provides a novel stereospecific route for synthesis of norephedrine type molecules.

### Objectives of the Invention

It is an objective of the present invention to provide a method to convert a α-hydroxy ketone (Formula I) to β-amino alcohol (Formula III) in the following manner as depicted below.

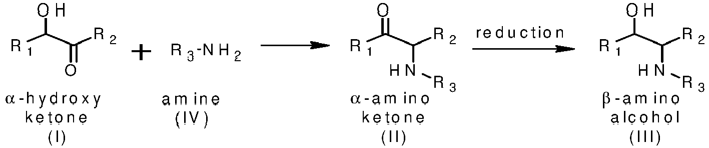

It is another objective of the invention to provide a process for preparation of β-amino alcohol (Formula III, R₃ = H) by reduction of the α-amino ketone [Formula II, R₄ = C1-8 alkyl; C4-6 cyclo alkyl, or -COOR₆ (R₆=C1-8 alkyl), R₅ = (substituted) aryl, under hydrogenative de-(aryl) alkylation conditions as illustrated in scheme II.

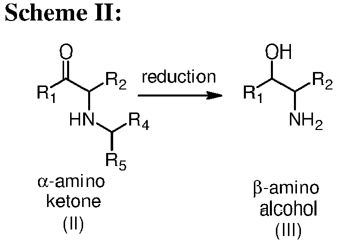

Yet another object of the present invention is to provide a method for preparation of norephedrine derivatives (Formula III, R1= Ph, (subst)Ph; R2=CH3) from Phenylacetylcarbinol (Formula I, R1=Ph; R2=CH3) by reaction with the chiral amine [Formula IV, R3 = R4R5CH- where, R4 = C1-8 alkyl, C4-6 cycloalkyl, or -COOR6 (R6=C1-8 alkyl); R5 = (substituted)aryl by first forming the α-amino ketone [Formula II, R1 = (subst)Ph, R2 = CH3, R4 = C1-8 alkyl, C4-6 cycloalkyl, or -COOR6 (R6=C1-8 alkyl); R5 = (substituted)aryl followed by removal of the R4R5CH- group by hydrogenolysis using Palladium catalysts.

It is yet another objective of the present invention to provide a method to prepare pure enantiomers of Norephedrine derivatives (Formula III, R₁= Ph, (subst)Ph; R₂=CH₃, either 1R,2S or 1S,2R isomers) by using appropriately substituted Phenylacetylcarbinol (Formula I, R₁= Ph, (subst)Ph; R₂=CH₃, R-isomer or S-isomer or racemic) by treatment with pure enantiomers of optionally ring substituted α-(methyl)benzylamine (Formula IV, R₃ = R₄R₅CH- where, R₄ = CH₃; R₅ = Ph, (subst)Ph, either pure S or pure R isomer).

### Summary of the Invention

According to an embodiment, the invention discloses a method of preparation of β-amino alcohol of formula (III)

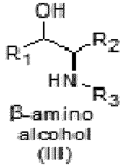

Wherein R1 is Ph, (substituted) Ph.

R₂ is C1-8 alkyl or C4-6 cyclo alkyl.

R₃ = R4R5CHNH2 where, R₄ = C1-8 alkyl, C4-6 cyclo alkyl, or - COOR6 (R6=C1-C8 alkyl); R₅ = (substituted) aryl.

The method including subjecting an α-hydroxy ketone of formula (I)

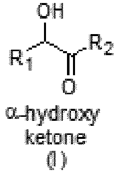

Wherein R₁ and R₂ are as defined above is reacted with a chiral amine of formula R₃NH₂ where R₃ = R₄R₅CH- where, R₄ = C1-8 alkyl, C4-6 cyclo alkyl, or -COOR6 (R6=C1-8 alkyl); R₅ = (substituted) aryl to produce a compound of formula (II)

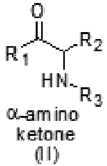

In which R₁, R₂ and R₃ are as defined above, followed by reduction to form the compound of formula (III).

The method may be represented by the reaction scheme below:

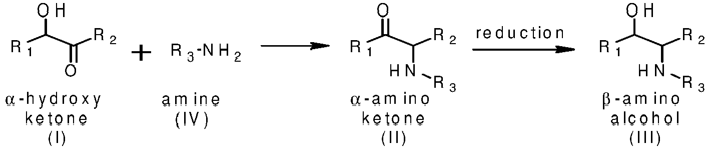

According to an embodiment of the invention, the reaction of α-hydroxy ketone of formula (I) with the chiral amine of formula (IV) is carried out in a solvent selected from a group consisting of C1-4 aliphatic alcohol, acetate esters of lower (C1-3) aliphatic alcohols and hydrocarbon solvents such as benzene, toluene, xylenes and monochlorobenzene.

### Detailed Description of the Invention

Reference will now be made in detail to the description of the present subject matter, one or more examples of which are shown in figures. Each example is provided to explain the subject matter and not a limitation.

In order to more clearly and concisely describe and point out the subject matter of the claimed invention, the following definitions are provided for specific terms which are used in the following written description.

By the term "*Beta amino alcohol*" we mean an organic compound that contains both an amine functional group and an alcohol functional group, as context requires.

By the term "*a-hydroxy ketone*" we mean a class of organic compounds that shares a common functional group consisting of a hydroxyl group placed on the α-position of a carbonyl group, as context requires.

By the term "*chiral amine*" we mean a compound which is enantiomerically pure, as context requires.

By the term "*Reduction reaction*" we mean a chemical reaction in which hydrogen is added to or oxygen is removed from a compound, as context requires.

By the term "*Hydrogenation reaction*" we mean a chemical reaction between molecular hydrogen (H2) and another compound or element, usually in the presence of a catalyst, as context requires.

By the term "*Hydrogenolysis*" we mean a chemical reaction whereby a carbon-carbon or carbon-heteroatom single bond is cleaved or undergoes "lysis" by hydrogen, as context requires.

In the method of the present invention α-hydroxy ketone (Formula I) is converted to β-amino alcohol (Formula III) in following steps:
a) α-hydroxy ketone (Formula I) is treated with a chiral amine (Formula IV) to give the α-amino ketone (Formula II) with a rearrangement.
b) The α-amino ketone is reduced to give β-amino alcohol (Formula III). The whole reaction is depicted below in Scheme III. 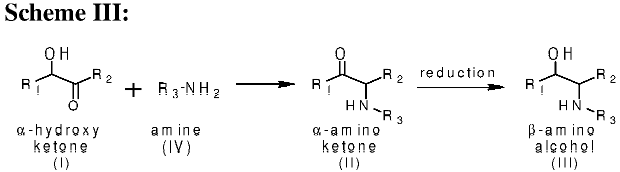
Where,
R₁ is Ph, (substituted) Ph. and R₂ is C1-8 alkyl or C4-6 cycloalkyl; R₃ = R₄R₅CHNH₂ where, R₄ = C1-8 alkyl, C4-6 cyclo alkyl, or -COOR₆ (R₆=C1-C8 alkyl); R₅ = (substituted) aryl.

The above reaction is carried out by dissolving the α-hydroxy ketone in a suitable solvent selected from a group comprising C1-4 aliphatic alcohol, acetate esters of lower (C1-3) aliphatic alcohols, lower alkyl esters such as ethyl acetate or isopropyl acetate, halogenated hydrocarbons such as ethylene dichloride, chloroform or methylene chloride, hydrocarbon solvents such as benzene, toluene, xylenes, monochlorobenzene, ethereal solvents such as diisopropyl ether, methyl t-butyl ether, di-n-butyl ether, 1,2-dimethoxy ethane, ethylene glycol monomethyl ether, diphenyl ether etc. and reacting with the amine (Formula IV, R₃ as above) optionally in the presence of dehydrating agents such as sodium sulphate, magnesium sulphate, calcium oxide or molecular sieves. The reaction can be carried out at a temperature between 0° and the boiling point of the solvent. The moisture formed in the reaction can also be removed by distillation from reaction mass. The α-hydroxyketone initially forms an imine compound, which undergoes rearrangement to more stable compound α-aminoketone as shown below in Scheme IV.

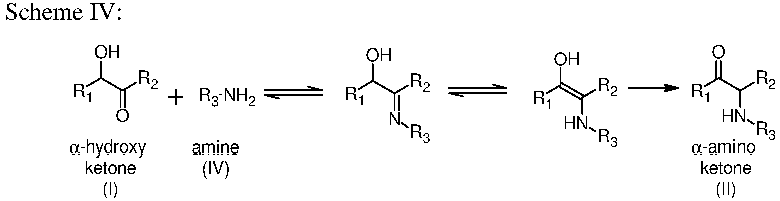

The α-amino ketone (Formula II) is obtained in a complete stereospecific manner as a single diastereomer. The stereospecificity of the reaction is controlled by the chirality of the chiral amine and not necessarily that of the α-hydroxy ketone. The configuration of the amino center in the predominant diastereomer of the amino ketone is dependent on the configuration of the original chiral amine. The α-amino ketone product may be isolated as an acid addition salt with inorganic or organic acids or may be taken in-situ for the next step of reduction.

According to the present invention, the α-amino ketone either as an isolated acid addition salt or in crude form from the previous step is reduced to β-amino alcohol by dissolving in a suitable solvent and treating with a reducing agent selected from a group comprising sodium borohydride, lithium borohydride, potassium borohydride to give N-substituted β-amino alcohol product. Alternatively, the α-amino ketone may be reduced by catalytic hydrogenation using Ni, or Pt catalysts to give N-substituted β-amino alcohol product. The product of such reduction methods may further be hydrogenated using palladium catalyst under hydrogenolysis conditions to get primary amine product. Alternatively, the α-amino ketone may be directly reduced by catalytic hydrogenation using Palladium catalysts to give the primary amine product.

In an embodiment of the invention, the starting α-hydroxy ketone has R₁ as a phenyl or a substituted phenyl group and R₂ as methyl. The substituents in the substituted phenyl is selected from halo (Cl, Br, I), hydroxy, methoxy, ethoxy, nitro, amino, substituted amino, which may be placed in ortho, meta or para positions with respect to the side-chain. In this embodiment, the chiral amine used is R3-NH2, [Formula IV, R₃ = R₄R₅CH- where, R₄ = H, C1-8 alkyl, C4-6 cycloalkyl, or -COOR₆ (R₆=C1-8 alkyl); R₅ = (substituted) aryl. The product of the reaction of α-hydroxy ketone with the amine IV, i.e. α-amino ketone, is isolated either as base or as acid addition salt. It is then hydrogenated using palladium catalyst to give β-amino alcohol (Formula III, R₁ and R₂ as above, R₃=H).

In another embodiment of the invention, the α-hydroxy ketone used is either S- or R- or dl-Phenylacetylcarbinol and the amine used is S- or R-α-methyl benzylamine. The product α-amino ketone obtained is 2(S)-[(αS-methyl benzyl) amino] propiophenone or 2(R)-[(αR-methyl benzyl) amino]propiophenone respectively, isolated as hydrochloride salts. The hydrochloride salts are hydrogenated over palladium catalyst to afford (1R, 2S)-2-amino-1-phenylpropan-1-ol (L-norephedrine) or (1S, 2R)-2-amino-1-phenylpropan-1-ol (D-norephedrine) isolated as hydrochlorides or as free base.

In yet another embodiment of the invention, the α-hydroxy ketone used is R-4-hydroxyphenylacetylcarbinol and the amine used is S- or R-α-methyl benzylamine. The product α-amino ketone obtained is 4'-hydroxy-2(S)-[(αS-methyl benzyl) amino] propiophenone or 4'-hydroxy-2(R)-[(aR-methyl benzyl) amino] propiophenone respectively, isolated as hydrochloride salts. The hydrochloride salts are hydrogenated over palladium catalyst to afford (1R,2S)-4'-hydroxy-2-amino-1-phenylpropan-1-ol (L-4-hydroxynorephedrine) or (1S,2R)-4'-hydroxy-2-amino-1-phenylpropan-1-ol (D-4-hydroxynorephedri-ne) isolated as hydrochloride salt or as free base.

In order that this invention to be more fully understood the following preparative and testing examples are set forth. These examples are for the purpose of illustration only and are not to be construed as limiting the scope of the invention in any way.

### Examplel:Preparation of 2(S)-[(αSmethyl benzyl)amino]propiophenone Hydrochloride

R-Phenylacetylcarbinol (50 g) is dissolved in 300 ml toluene. It is then mixed with S-α-methyl benzylamine (47 g) and 2 g of anhydrous magnesium sulphate and stirred at ambient temperature for 4 hours. The drying agent is filtered off, mixture is acidified with aq. hydrochloric acid. The mixture is refluxed under Dean-Stark apparatus until there is complete removal of water. The mixture is then cooled and filtered. 2(S)-[(αS-methyl benzyl) amino] propiophenones Hydrochloride is obtained as product.

### Example 2: Preparation of 2(R)-[(αR methyl benzyl)amino] propiophenone Hydrochloride

R-Phenylacetylcarbinol (50 g) is dissolved in 300 ml toluene. It is then mixed with R-α-methylbenzylamine (47 g) and 2 g of anhydrous magnesium sulphate and stirred at ambient temperature for 4 hours. The drying agent is filtered off, mixture is acidified with aq. hydrochloric acid. The mixture is refluxed under Dean-Stark apparatus until there is complete removal of water. The mixture is then cooled and filtered. 2(R)-[(αR-methyl benzyl) amino] propiophenone Hydrochloride is obtained as product.

### Example 3: Preparation of 4'-Hydroxy-2(S)-[(αS-methyl benzyl)amino] propiophenone Hydrochloride

R-4'-Hydroxyphenylacetylcarbinol (50 g) is dissolved in 300 ml toluene. It is then mixed with S-α-methyl benzylamine (47 g) and 2 g of anhydrous magnesium sulphate and stirred at ambient temperature for 4 hours. The drying agent is filtered off, mixture is acidified with aq. hydrochloric acid. The mixture is refluxed under Dean-Stark apparatus until there is complete removal of water. The mixture is then cooled and filtered. 4'-Hydroxy-2(S)-[(αS-methyl benzyl) amino] propiophenone Hydrochloride is obtained as product.

### Example 4: Preparation of 4'-Hydroxy-2(R)-[(αR-methyl benzyl) amino] propiophenone Hydrochloride

R-4'-Hydroxyphenylacetylcarbinol (50 g) is dissolved in 300 ml toluene. It is then mixed with R-□-methyl benzylamine (47 g) and 2 g of anhydrous magnesium sulphate and stirred at ambient temperature for 4 hours. The drying agent is filtered off, mixture is acidified with aq. hydrochloric acid. The mixture is refluxed under Dean-Stark apparatus until there is complete removal of water. The mixture is then cooled and filtered. 4'-Hydroxy-2(R)-[(αR-methyl benzyl) amino] propiophenone Hydrochloride is obtained as product.

### Example 5: Preparation of (1R, 2S)-2-amino-1-phenylpropan-1-ol (L-norephedrine) Hydrochloride

50 g of 2(S)-[(αS-methyl benzyl)amino] propiophenone Hydrochloride is dissolved in 300 ml of methanol, hydrogenated over 10 g of 5% Pd-C catalyst at pressure 4-5 kg/cm2 and temperature 50-70°C. After hydrogen absorption ceases, the catalyst is filtered and product L-norephedrine hydrochloride is recovered by solvent evaporation. Melting point: 172-174°, specific optical rotation: -33.5° (5% in water), chiral purity: 99.5% ee.

### Example 6: Preparation of (IS, 2R)-2-amino-1-phenylpropan-1-ol (D-norephedrine) Hydrochloride

50 g of 2(R)-[(αR-methyl benzyl)amino]propiophenone Hydrochloride is dissolved in 300 ml of methanol, hydrogenated over 10 g of 5% Pd-C catalyst at pressure 4-5 kg/cm² and temperature 50-70°C. After hydrogen absorption ceases, the catalyst is filtered and product D-norephedrine hydrochloride is recovered by solvent evaporation. Melting point: 171-173°, specific optical rotation: +32.9° (5% in water), chiral purity: 99.2% ee.

### Example 7: Preparation of (1R,2S)-4'-Hydroxy-2-amino-1 phenylpropan-1-ol(L-4-Hydroxynorephedrine

50 gm of 4'-Hydroxy-2(S)-[(αS-methyl benzyl)amino]propiophenone Hydrochloride is dissolved in 300 ml of methanol, hydrogenated over 10 g of 5% Pd-C catalyst at pressure 4-5 kg/cm² and temperature 50-70°C. After hydrogen absorption ceases, the catalyst is filtered. Methanol is evaporated off. Residue is diluted with 100 ml water and 6.5 g of sodium hydroxide is added. The product (1R, 2S)-4'-Hydroxy-2-amino-1-phenylpropan-1-ol (L-4-Hydroxynorephedrine) precipitates. It is isolated by filtration washing with water to get rid of excess of alkali. Melting point: 158-160°, specific optical rotation: -18.5° (2% in methanol), chiral purity: 99.8% ee.

### Example 8: Preparation of (1S, 2R)-4'-hydroxy-2-amino-1-phenylpropan-1-ol (D-4-Hydroxynorephedrine)

50 gm of 4'-Hydroxy-2(R)-[(αR-methyl benzyl)amino]propiophenone Hydrochloride is dissolved in 300 ml of methanol, hydrogenated over 10 g of 5% Pd-C catalyst at pressure 4-5 kg/cm² and temperature 50-70°C. After hydrogen absorption ceases, the catalyst is filtered. Methanol is evaporated off. Residue is diluted with 100 ml water and 6.5 g of sodium hydroxide is added. The product (1S, 2R)-4' -Hydroxy-2-amino-1-phenylpropan-1 -ol (D-4-Hydroxynorephedrine) precipitates. It is isolated by filtration washing with water to get rid of excess of alkali. Yield: Melting point: 160-161°, specific optical rotation: -17.9° (2% in methanol), chiral purity: 99.3% ee.

### Example 9: Preparation of (1R, 2S)-1-phenyl-2-{[(1S)-1-phenylethyl]amino}propan-1-ol Hydrochloride

50 g of 2(S)-[(αS-methyl benzyl)amino]propiophenone Hydrochloride is dissolved in 300 ml of methanol, hydrogenated over 10 g of Raney Ni catalyst at pressure 4-5 kg/cm² and temperature 50-70°C. After hydrogen absorption ceases, the catalyst is filtered and product (1R, 2S)-1-phenyl-2-{[(1S)-1-phenylethyl] amino} propan-1-ol Hydrochloride is recovered by solvent evaporation and crystallization from ethanol: ether. Melting point: 212-215°, specific optical rotation: -30.5° (5% in water), HPLC purity: R, S, S-isomer: 99.65%; S, S, S-isomer: 0.15%.

### Example 10: Preparation of methyl (2S)-{[(2S)-1-oxo-1-phenylpropan-2-yl]amino}(phenyl) ethanoate Hydrochloride

R-Phenylacetylcarbinol (50 g) is dissolved in 300 ml toluene. It is then mixed with S-α-phenylglycine methyl ester (55 g) and 2 g of anhydrous magnesium sulphate and stirred at ambient temperature for 16 hours. The drying agent is filtered off, mixture is acidified with aq. hydrochloric acid. The mixture is refluxed under Dean-Stark apparatus until there is complete removal of water. The refluxing is continued for 16 hours after removal of water. The mixture is then cooled and filtered. Methyl (2*S*)-{[(2*S*)-1-oxo-1-phenylpropan-2-yl] amino} (phenyl) ethanoate HCl is obtained as product.

### Example 11: Preparation of L-norephedrine HCl from methyl (2S)-{[(2S)-1-oxo-1-phenylpropan-2-yl]amino}(phenyl) ethanoate Hydrochloride

50 g of methyl (2*S*)-{[(2*S*)-1-oxo-1-phenylpropan-2-yl]amino}(phenyl) ethanoate Hydrochloride is dissolved in 150 ml of methanol and hydrogenated over 1.5 g of 10% palladium on charcoal catalyst at 6 kg/cm² hydrogen pressure until gas absorption ceases. The catalyst is filtered, methanol evaporated off and residue crystallized from acetone. Melting point: 172-174°, specific optical rotation: - 34.3° (5% in water), chiral purity: 99.15% ee.

## Claims

1. A method for the preparation of β-amino alcohol of 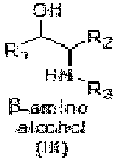 formula (III):
in which
R₁ is Ph, substituted Ph,;
R₂ is C1-8 alkyl or C4-6 cyclo alkyl;
R₃ = R₄R₅CHwhere, R₄ = C1-8 alkyl, C4-6 cyclo alkyl, or -COOR6 R6=C1-C8 alkyl; R₅ = (substituted) aryl
the method including subjecting a α-hydroxy ketone of 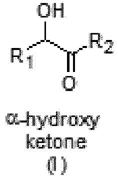 formula (I):
in which R₁ and R₂ are as defined above is reacted with a chiral amine of formula R₃NH₂ where R₃ = R₄R₅CH- where, R₄ = C1-8 alkyl, C4-6 cycloalkyl, or -COOR6 R₆=C1-8 alkyl; R₅ = (substituted) aryl to produce a compound of 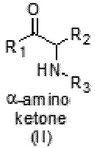 formula (II) in which R₁, R₂ and R₃ are as defined above, followed by reduction to form the compound of formula (III).

2. The method as claimed in claim 1, wherein the reaction of said α-hydroxy ketone of formula (I) with the chiral amine of formula (IV) is carried out in a solvent selected from a group consisting of C1-4 aliphatic alcohol, acetate esters of lower (C1-3) aliphatic alcohols and hydrocarbon solvents such as benzene, toluene, xylenes and monochlorobenzene.

3. The method as claimed in claim 1, wherein said α-amino ketone of formula (II) is isolated as such or as an acid addition salt with inorganic or organic acids.

4. The method as claimed in claim 1, wherein said α-hydroxy ketone formula (I) is selected from a group consisting of S-Phenylacetylcarbinol, R-Phenylacetylcarbinol, dl-Phenylacetylcarbinol, S-4-hydroxy Phenylacetylcarbinol, R-4-hydroxy Phenylacetylcarbinol and dl-4-hydroxy Phenylacetylcarbinol.

5. The method as claimed in claim 1, wherein said chiral amine of formula (IV) is either S-α-methyl benzylamine or R-α-methyl benzylamine.

6. The method as claimed in claim 1, wherein the reaction between said α-hydroxy ketone of formula (I) and the chiral amine of formula (IV) is carried out at a temperature between 0°C and the boiling point of the solvent.

7. The method as claimed in claim 1, wherein the reduction of said α-amino ketone of formula (II) is carried out using reducing agents selected from a group consisting of sodium borohydride, lithium borohydride, potassium borohydride and lithium aluminium hydride.

8. The method as claimed in claim 1, wherein the reduction of said α-amino ketone of formula (II) is carried out using catalytic hydrogenation at pressure range between atmospheric pressure and 20 kg/cm2 over a catalyst selected from a group consisting of raney nickel, platinum dioxide and platinum on carbon.

9. The method as claimed in claim 8 wherein the loading of said platinum on carbon is between 1 and 20% w/w.

10. The method as claimed in claim 8, wherein the temperature of hydrogenation reaction is between ambient and 100°C.

## Patentansprüche

1. Verfahren zur Zubereitung von β-Amino-Alkohol der 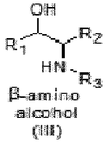 Formel (III):
in der
R₁ Ph ist, substituiertes Ph.;
R₂ C1-8-Alkyl oder C4-6-Cyclo-Alkyl ist;
R₃ = R₄R₅CH ist, wobei R₄ = C1-8-Cyclo-Alkyl ist oder -COOR6 R6 = C1-C8-Alkyl ist; R₅ = (substituiertes) Aryl ist
wobei das Verfahren das Unterziehen eines α-Hydroxyketons der 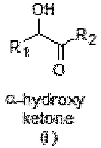 der Formel (I) enthält:
in der R₁ und R₂, die wie oben definiert sind, mit einem chiralen Amin der Formel R₃NH₂ zum Reagieren gebracht werden,
wobei R₃ = R₄R₅CH-, wobei R4 = C1-8-Alkyl ist, C4-6 Cycloalkyl ist oder -COOR6 R₆ = C1-8-Alkyl ist; R₅ = (substituiertes) Aryl zum Produzieren eines Bestandteils von 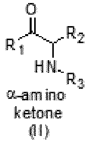 der Formel (II) ist
wobei R₁, R₂ und R₃ wie oben definiert sind, gefolgt von einer Reduktion zum Bilden des Bestandteils der Formel (III).

2. Verfahren gemäß Anspruch 1, wobei die Reaktion des genannten α-Hydroxyketons der Formel (I) mit dem chiralen Amin der Formel (IV) in einem Lösungsmittel durchgeführt wird, das aus einer Gruppe ausgewählt wird, bestehend aus C1-4 aliphatischem Alkohol, Acetatestern der unteren (C1-3) aliphatischen Alkohole und Kohlenwasserstoff-Lösungsmitteln, wie z. B. Benzol, Toluol, Xylenen und Monochlorbenzol.

3. Verfahren gemäß Anspruch 1, wobei das genannte α-Aminoketon der Formel (II) als solches oder als ein saures zusätzliches Salz mit anorganischen oder organischen Säuren isoliert wird.

4. Verfahren gemäß Anspruch 1, wobei das genannte α-Hydroxyketon der Formel (I) aus einer Gruppe ausgewählt wird, bestehend aus S-Phenylacetylcarbinol, R-Phenylacetylcarbinol, dl-Phenylacetylcarbinol, S-4-Hydroxy-Phenylacetylcarbinol, R-4-Hydroxy-Phenylacetylcarbinol und dl-4-Hydroxy-Phenylacetylcarbinol.

5. Verfahren gemäß Anspruch 1, wobei das genannte chirale Amin der Formel (IV) entweder S-α-Methyl-Bentylamin oder R-α-Methyl-Bentylamin ist.

6. Verfahren gemäß Anspruch 1, wobei die Reaktion zwischen dem genannten α-Hydroxyketon der Formel (I) und das chirale Amin der Formel (IV) bei einer Temperatur zwischen 0° C und dem Siedepunkt des Lösungsmittels ausgeführt wird.

7. Verfahren gemäß Anspruch 1, wobei die Reduktion des genannten α-Aminoketons der Formel (II) unter Verwenden von Reduktionsmitteln ausgeführt wird, die aus einer Gruppe ausgewählt sind, bestehend aus Natrium-Borhydrid, Lithium-Borhydrid, Kalium-Borhydrid und Lithium-Aluminiumhydrid.

8. Verfahren gemäß Anspruch 1, wobei die Reduktion des genannten α-Aminoketons der Formel (II) unter Verwenden einer katalytischen Hydrogenierung in einem Druckbereich zwischen dem atmosphärischen Druck und 20 kg/cm2 über einem Katalysator ausgeführt wird, der aus einer Gruppe ausgewählt wird, bestehend aus Raneynickel, Platindioxid und Platin auf Kohlenstoff.

9. Verfahren gemäß Anspruch 8, wobei das Laden des genannten Platins auf den Kohlenstoff zwischen 1 und 20 Gew.-% beträgt.

10. Verfahren gemäß Anspruch 8, wobei die Temperatur der Hydrogenierungsreaktion zwischen der Raumtemperatur und 100° C beträgt.

## Revendications

1. Procédé de préparation d'un β-aminoalcool de formule (III) 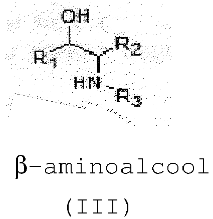 dans laquelle
R₁ est un Ph, un Ph substitué ;
R₂ est un alkyle en C1-8 ou un cycloalkyle en C4-6 ;
R₃ = R₄R₅CH où R₄ = alkyle en C1-8, cycloalkyle en C4-6, ou bien -COOR₆ R₆ = alkyle en C1-8 ;
R₅ = aryle (substitué) ;
ledit procédé comprenant le fait de soumettre une α-hydroxycétone de formule (I), 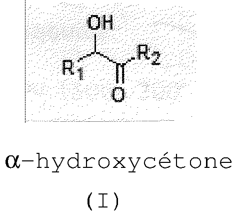 dans laquelle
R₁ et R₂ sont telles que définies ci-dessus, à une réaction avec une amine chirale de formule R₃NH₂, où R₃ = R₄R₅CH, où R₄ = alkyle en C1-8, cycloalkyle en C4-6 ou bien -COOR₆ R₆ = alkyle en C1-8 ; R₅= aryle (substitué) afin de produire un composé de formule (II) 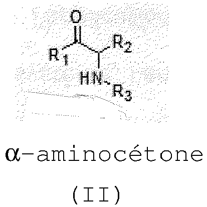 dans laquelle R₁, R₂ et R₃ sont telles que définies ci-dessus, ladite réaction étant suivie d'une réduction afin de former le composé de formule (III).

2. Procédé selon la revendication 1, dans lequel la réaction de ladite α-hydroxycétone de formule (I) avec l'amine chirale de formule (IV) est effectuée dans un solvant sélectionné parmi un groupe se composant d'alcool aliphatique en C1-4, d'esters d'acétate d'alcools aliphatiques inférieurs (en C1-3) et de solvants d'hydrocarbures tels que du benzène, du toluène, des xylènes et du monochlorobenzène.

3. Procédé selon la revendication 1, dans lequel ladite a-aminocétone de formule (II) est isolée en tant que telle ou en tant que sel d'addition d'acide avec des acides inorganiques ou organiques.

4. Procédé selon la revendication 1, dans lequel ladite α-hydroxycétone de formule (I) est sélectionnée parmi un groupe se composant de S-phénylacétylcarbinol, de R-phénylacétylcarbinol, de d1-phénylacétylcarbinol, de S-4-hydroxy phénylacétylcarbinol, de R-4-hydroxy phénylacétylcarbinol et de d1-4-hydroxy phénylacétylcarbinol.

5. Procédé selon la revendication 1, dans lequel ladite aminé chirale de formule (IV) est soit de la S-α-méthylbenzylamine soit de la R-α-méthylbenzylamine.

6. Procédé selon la revendication 1, dans lequel la réaction entre ladite α-hydroxycétone de formule (I) et l'amine chirale de formule (IV) est effectuée à une température comprise entre 0°C et le point d'ébullition du solvant.

7. Procédé selon la revendication 1, dans lequel la réduction de ladite α-aminocétone de formule (II) est effectuée en utilisant des agents réducteurs sélectionnés parmi un groupe se composant de borohydrure de sodium, de borohydrure de lithium, de borohydrure de potassium et d'hydrure d'aluminium et de lithium.

8. Procédé selon la revendication 1, dans lequel la réduction de ladite α-aminocétone de formule (II) est mise en œuvre en utilisant une hydrogénation catalytique effectuée à une plage de pression comprise entre la pression atmosphérique et une pression de 20 kg/cm² appliquée à un catalyseur sélectionné parmi un groupe se composant de nickel de Raney, de dioxyde de platine et de platine sur du carbone.

9. Procédé selon la revendication 8, dans lequel la charge dudit platine sur du carbone est comprise entre 1 % p/p et 20 % p/p.

10. Procédé selon la revendication 8, dans lequel la température de réaction d'hydrogénation est comprise entre la température ambiante et 100°C.
